Europäisches Patentamt

European Patent Office

Office européen des brevets

(11) Publication number: **0 085 289**

**A1**

# (12) EUROPEAN PATENT APPLICATION

(21) Application number: **82870080.7**

(22) Date of filing: **20.12.82**

(51) Int. Cl.³: **C 07 C 37/08**
**C 07 C 45/53, C 07 C 39/04**
**C 07 C 49/08**

(30) Priority: **24.12.81 US 334411**

(43) Date of publication of application:
**10.08.83 Bulletin 83/32**

(84) Designated Contracting States:
**BE DE FR GB IT NL SE**

(71) Applicant: **MONSANTO COMPANY**
**Patent Department 800 North Lindbergh Boulevard**
**St. Louis, Missouri 63166(US)**

(72) Inventor: **Canfield, Robert Caldwell**
**1205 Terrace Drive**
**Alvin Texas 77511(US)**

(72) Inventor: **Dupree, Louis Edward, Jr.**
**8101 Woodacres Drive**
**Hitchcock Texas 77563(US)**

(72) Inventor: **Hunt, Charles Ronald**
**730 Seacliff Street**
**Houston Texas 77062(US)**

(74) Representative: **McLean, Peter et al,**
**Monsanto Europe S.A. Patent Department Avenue de**
**Tervuren 270-272 Letter Box No 1**
**B-1150 Brussels(BE)**

(54) **Process for direct neutralization of product mixture resulting from acid catalyzed cleavage of alkyl aromatic hydroperoxides.**

(57) The neutralization of product mixture resulting from acid catalyzed cleavage of alkyl aromatic hydroperoxides using metal hydroxides in a form sufficient to maintain the water content of the product mixture at less than about 6% by weight during neutralization with phenol the preferred product and sodium hydroxide the preferred hydroxide.

EP 0 085 289 A1

20-19-9123  **0085289**

## PROCESS FOR DIRECT NEUTRALIZATION OF PRODUCT MIXTURE RESULTING FROM ACID CATALYZED CLEAVAGE OR ALKYL AROMATIC HYDROPEROXIDES

### BACKGROUND OF THE INVENTION

This invention relates to an improvement in the manufacture of hydroxy aromatic compounds by acid catalyzed decomposition, rearrangement or cleavage of alkyl aromatic hydroperoxides and removal of the inroganic acid catalysts and, in particular, to a water control neutralization process wherein substantially non-aqueous metal hydroxide solutions are used to neutralize the acid catalyst and water addition is subject to close control. Neutralization and removal of the acid catalyst is desirable in order to prevent the catalyst from catalyzing further reactions resulting in the production of undesirable by-products and to reduce corrosion of the recovery system.

### DISCUSSION OF THE PRIOR ART

In the prior art, the crude product mixture from the acid catalyzed decomposition of alkyl aromatic hydroperoxides has been neutralized by a variety of neutralizing agents such as, for example, ammonia and other gases in U.S. 3,927,124 to Burkholder, alkali and alkaline earth metal carbonates in U.S. 3,376,352 to Domenicali et al, and by hydroxides in U.S. 2,734,085 to Adams et al. These neutralization methods cause precipitation of large amounts of inorganic salts with the organic product that are difficult to remove.

The acid catalyst has also been removed by washing with water or salt solutions as in U.S. 2,951,870 to Cooke. A major disadvantage of such washing methods is that the organic phase of the crude reaction product mixture becomes saturated with water. Since both acetone and phenol, two of the more commonly desired products, have substantial water solubility, the aqueous wash retains a significant quantity of organic product. Therefore, removal of the acid catalyst by an aqueous wash results in a yield loss and increase in waste load on water treating facilities as well as increased energy requirements in purification. Furthermore, the inorganic salts dissolved in the organic phase are concentrated in the residual heavy tars and cause a serious problem where the tars are to be burned for fuel.

It has now been found that the acid catalyst can be removed by addition of a limited amount of metal hydroxide to the crude product mixture under pH control and low water content. By careful control of conditions an organic product is produced which contains less than about 6 wt % and preferably less than 3 wt % water, less than about 100 ppm inorganic salts and essentially no acidic catalyst.

## SUMMARY OF THE INVENTION

The invention involves a process for neutralization of the acid catalyst from the crude reaction product mixture produced by acid catalyzed rearrangement of alkyl aromatic hydroperoxides characterized by adding a small amount of a metal hydroxide neutralizing agent wherein the metal is selected from lithium, sodium, potassium, rubidium, cesium, calcium, strontium and barium to the crude product mixture in a form sufficient to maintain the water content of the mixture at less than about 6 wt % and removing the resultant salt of the acid as formed.

Additionally, the invention relates to a process for the production of alkyl aromatic hydroperoxide rearrangement products comprising cleaving alkyl aromatic hydroperoxides by heating in the presence of an inorganic acid catalyst to produce a crude product mixture characterized by adding to said mixture a neutralizing amount of a metal hydroxide neutralizing agent wherein the metal is selected from lithium, sodium, potassium, rubidium, cesium, calcium, strontium and barium, said neutralizing agent being optionally dissolved in neutralized product mixture, controlling the pH of the crude product mixture between about 50°C and the boiling point of the mixture, maintaining the water content of the crude product mixture at below 6% by wt, removing the resultant acid salt as formed and separating the rearrangement products from the neutralized crude product mixture.

## DETAILED DESCRIPTION OF THE INVENTION

An important aspect of the neutralization process of the invention is that by careful control of the amount of water in the neutralizing agent and the conditions during neutralization the organic phase of the crude reaction product mixture will contain less than about 6 wt % and preferably less than 3 wt % water, less than about 100 ppm inorganic salts and essentially no acid catalyst.

In maintaining the amount of water present during neutralization at less than about 6 wt % there are a number of possible sources of water to be considered. The crude cleavage reaction product contains about 1.0 wt % up to a maximum of about 2.5 wt % water from the reactants and the cleavage reaction. The exact amount of water in the crude product is easily determined by a water analyzer or other suitable means. Additionally, water is usually introduced into the mixture to remove the sodium sulfate formed, as a result of neutralization, from the mixture. Current cleavage processes frequently included treating of hydroxyacetone by-product with caustic which causes sodium phenate to be formed. This sodium phenate is recycled to recover product phenol by acid neutralization producing sodium salts such as sodium sulfate. Neutralization of the crude product mixture according to the prior art thus includes a large amount of added water, about 6-10 wt % for removal of sodium sulfate. Two liquid phases are present in the crude mixture, an organic liquid phase containing 10-14 wt % water, and an aqueous sodium sulfate rich brine phase. Circulating sodium sulfate brine is used as the neutralizing agent to extract sulfuric acid from the organic phase which contains 8-14 wt % water and about 300-800 ppm sodium sulfate.

The neutralization process of this invention avoids introduction of substantial amounts of additional water into the crude product mixture from the neutralization agent and there is no aqueous sodium sulfate brine phase. By maintaining the water content at below 6 wt % and preferably between about 1 and 4% by wt in the crude product mixture, a liquid organic phase and a solid phase will result from the neutralization. The phase separation caused by direct addition of essentially non-aqueous concentrated neutralizing agent enables separating an organic phase containing less than about 100 ppm sodium sulfate. Most of the sodium sulfate has precipitated in the solid phase. After neutralization, the water content of the organic phase is less than about 6% and most preferably less than 3% water. Any substantial amounts of water in excess of about 6% by weight will cause additional quantities of inorganic salts to be carried forward in the organic product and is thus to be avoided.

In order to minimize water concentration and facilitate the said catalyst removal from the crude product mixture, the neutralizing agent is utilized in any convenient low water containing form such as the anhydrous base, a concentrated aqueous solution or a concentrated aqueous solution thereof that has been diluted by a nonaqueous solvent. By using the neutralizing agent in such form the amount of water added to the crude product mixture will not exceed about 3% by wt thus resulting in a total water content of less than about 6 wt % being present during neutralization. The salt formed as a result of such neutralization precipitates and is readily separated by any suitable means. While any type of inert non-aqueous solvent which is miscible in the reaction mixture may be used, it is preferred that the hydroxide be dissolved in neutralized acid-free reaction product mixture such as a neutralized phenol-acetone mixture. After dissolution in the solvent, the concentration of hydroxide is preferably about one-fourth to four times the normality of the acid catalyst in the crude product mixture and most preferably of approximately the same equivalent concentration as that of the acid catalyst. A typical concentration range for the acid catalyst in the mixture is from about 0.10 to 0.5 wt %.

The crude product mixture to which the invention relates consists of a mixture of cleavage products and by-products. For example, acid catalyzed cleavage of cumene hydroperoxide usually produces a mixture of phenol and acetone products, acid catalyst, water and by-products such as α-methyl styrene, hydroxyacetone and heavy tars.

Any of the alkyl aromatic hydroperoxides may be subjected to acid catalyzed cleavage to form the crude product mixture used in the practice of the invention. For example, cumene hydroperoxide, also known as isopropylbenzene hydroperoxide, from which phenol is produced, p-di-isopropylbenzene hydroperoxide which yields hydroquinone and m-di-isopropylbenzene hydroperoxide from which resorcinol is derived may be used.

The temperature of the crude reaction mixture during neutralization may vary from about $20^{o}C$ to the boiling point of the mixture. The solid precipitate is preferably and much more easily separable from the liquid if the neutralization is done in excess of $50^{o}C$ up to the boiling point of the mixture.

Although sulfuric acid is the catalyst of choice in the alkyl aromatic hydroperoxide cleavage reaction, the neutralization method of the invention applies as well to any of the other inorganic acids, for example, arsenic, bromic, iodic, phosphoric, selenic and sulfonic which form water soluble salts and which may be used in the cleavage reaction.

The quantity of neutralizing agent to be used is that amount sufficient to neutralize the amount of acid catalyst present in the reaction mixture and is easily controlled by pH, a measurable quantity which allows precise control. The pH is controlled within the range of between about 2 and 7, preferably between about 3 and 5, and within this range the product desirably contains less than about 100 ppm ash (as determined by common combustion analysis techniques). As the pH of the reaction mixture is normally about 0.5 to 1.0, an increase in pH is required to effect neutralization.

Since an accurate measurement of the pH is a desirable part of the control of this process, sufficient water should be present for ionization to occur and is present as a normal process constituent, but may be added if necessary. About 1 wt % is usually sufficient. The amount is not critical since water has negligible effect on the pH of the reaction mixture.

Neutralization of the crude product mixture involves selection of one or more of the metal hydroxide neutralizing agents enumerated herein which may be used in solid form, as a concentrated aqueous solution or diluted with a compatable non-aqueous solvent, preferably acid-free product, to give a solution of hydroxide which is then ready to be added to the crude product mixture. Sodium hydroxide is preferred.

The invention is further illustrated by the following examples in which all parts and percents are by weight unless otherwise indicated.

In the examples the neutralization method of the invention is used in the sulfuric acid catalyzed cleavage of cumene hydroperoxide wherein sulfuric acid and cumene hydroperoxide are reacted and the crude reaction mixture flows to a mixing vessel where the sodium hydroxide neutralizing agent is added directly to the product mixture. A brine phase is not formed, instead a solid precipitate of sodium sulfate is formed and may be removed downflow using conventional solid/liquid separation procedures such as filtration, settling or centrifugation. The neutralizing agent is added preferably as a solution in neutralized product mixture diluted to approximately the concentration of the acid in the mixture. After removal of the sodium sulfate salt the remaining product mixture, now primarily an organic phase, is ready for further processing such as distillation where acetone, water and other volatile compounds may be recovered. The liquid fraction goes to additional distillation columns for further refinement and separation.

EXAMPLE I    Continuous Neutralization with Sodium Hydroxide

The neutralization is carried out in a single, well mixed vessel. Average residence time in the vessel is 20 min. 0.5 gpm (gallons per minute) of a crude reaction mixture containing 40 wt % phenol, 45 wt % acetone, 2.0 wt % water, 0.2 wt % $H_2SO_4$ catalyst and a blend of by-products consisting primarily of acetophenone, cumene,

hydroxyacetone and α-methyl styrene is added to the neutralizer vessel at 63°C. A pump is used to circulate neutralizer contents through an external mixing loop at about 5 gpm. A pH control loop is used to control addition of 25 wt % aqueous sodium hydroxide neutralizer to this external mixing loop such that the pH is held at 3.5. Neutralized product containing 0.36 wt % sodium sulfate gravity flows to a settling vessel. Clarified overflow from this settler was found to contain 130 ppm (parts per million) sodium sulfate and 2.5 wt % water. After passage through a deep media sand filter, sodium sulfate content in the phenol-acetone product was 50 ppm.

The bottoms from the settler vessel was concentrated to 1.4 wt % sodium sulfate. This slurry was further concentrated in an imperforate basket centrifuge to yield a centrate containing 100 ppm sodium sulfate and a cake containing 26 wt % sodium sulfate. The centrate stream is added to the feed to the sand filter.

The salt cake is mixed with water at 60°C to separate salt from organic components. A two phase system is formed consisting of a sodium sulfate brine (∼25 wt %) and an organic phase rich in phenol and acetone. This organic phase contains 11 wt % water and 460 ppm dissolved sodium sulfate. Adding this small stream back to the filter product has negligible effect on the total water or salt content.

EXAMPLE II   Distillation of Crude Neutralized Product

The cleavage product from Example I is distilled through a conventional 40-tray column. The column operates continuously under the following conditions:

| | |
|---|---|
| Pressure | $2.1 \text{ Kg/cm}^2$ |
| D/F (distillate to feed) | 0.67 mole/mole |
| External Reflux Ratio | 0.81 |
| Tray Efficiency | 75% |

- 8 -

Feed and product compositions were (wt %):

|  | Feed | Overhead | Bottoms |
|---|---|---|---|
| Acetone | 45.6 | 87.6 | 0 |
| Water | 3.0 | 5.6 | 0 |
| Hydroxyacetone | 1900 ppm | 3650 ppm | 30 |
| Phenol | 45.3 | .11 | 94.0 |
| Others | 5.9 | 6.0 | 6.0 |

EXAMPLE III   Neutralization with Concentrated Potassium Hydroxide

927 grams of crude cleavage product as described in Example I was neutralized to pH 4.0 by dropwise addition of 25 wt % KOH. After filtration through a 10 micron fritted filter, the product was found to contain 38 ppm potassium sulfate and less than 3 wt % water.

EXAMPLE IV   Neutralization with Dilute Potassium Hydroxide

25 wt % aqueous KOH is diluted to 2.0 wt % with neutralized, filtered cleavage product from Example I. Crude, acidic cleavage product was neutralized at $62^{o}$C using this solution. Addition was stopped at pH 4.0. After filtration, the product was found to contain 71 ppm potassium sulfate and less than 3 wt % water.

EXAMPLE V   Neutralization with Alkaline Earth Metals

Calcium and barium hydroxides and, for comparison, a non-effective alkaline earth metal, magnesium hydroxide, are combined with sufficient filtered, neutralized Example I cleavage product to give 0.2 mole % solutions. $Mg(OH)_2$ is only slightly soluble; $Ca(OH)_2$ and $Ba(OH)_2$ are substantially dissolved. 25 ml of acidic cleavage product as described in Example I is treated dropwise with the hydroxide solutions until a pH of 4.5 is reached. The $Ca(OH)_2$ and $Ba(OH)_2$ solutions were effective and easily controlled the pH at the 4.5 level. After centrifugation, the individual products were found to contain 282 ppm $CaSO_4$ and 9 ppm $BaSO_4$.

- 9 -

EXAMPLE VI    Sodium Hydroxide Neutralization with Higher Water
              Concentrate

The continuous process described in Example I was operated with higher (3.0 wt %) water content in the neutralizer feed. After centrifugation, the clarified organic product was found to contain 3.6 wt % water and 49 ppm ash (determined as sodium sulfate).

EXAMPLE VII    Neutralization at Various Water Concentrations

The continuous process of Example I was operated with various wt % water levels and the results were as follows:

| Wt % $H_2O$ | Ash Content in ppm |
|---|---|
| 6.0 | 104 |
| 8.0 | 136 |

There are a number of advantages to this neutralization procedure. After neutralization of the reaction mixture, an organic product rich phase and a solid phase are formed. The neutralized product is free of acid and since almost no additional water has been added, total removal of the water present in the mixture is easily accomplished in the first distillation column. This results in a major energy savings in phenol refining. Less than 100 ppm ash dissolved in the product permits the residual distillation tars to be directly utilized as a supplemental fuel without additional processing. Separation of crude product stream from the neutralized product mixture, since it contains low levels of water and dissolved inorganic salts, is easily accomplished by known procedures and product purification is simplified and more energy efficient relative to previously described processes.

While the foregoing examples relate to phenol and acetone crude reaction mixtures, the invention is not to be considered as restricted to neutralization of only such mixtures. It will be obvious to those skilled in the art that any acid catalyzed cleavage reaction mixtures can be neutralized by the process of this invention. Accordingly, it is intended that all modifications and variations which reasonably fall within the scope of the appended claims are included herein.

WE CLAIM:

1.    The process for neutralization of the crude reaction product mixture produced by the cleavage of alkyl aromatic hydroperoxides by heating in the presence of an inorganic acid catalyst characterized by adding to the crude product mixture a neutralizing amount of a metal hydroxide selected from lithium, sodium, potassium, rubidium, cesium, calcium, strontium and barium in a form sufficient to maintain the amount of water present in the mixture at less than about 6% by wt and removing the resultant precipitated salt of the acid as formed.

2.    The process of Claim 1 wherein the inorganic acid catalyst is sulfuric acid.

3.    The process of Claim 1 wherein the crude product mixture consists essentially of phenol, acetone, sulfuric acid, water and by-products.

4.    The process of Claim 1 wherein the metal hydroxide is selected from lithium hydroxide, sodium hydroxide, potassium hydroxide, barium hydroxide and calcium hydroxide.

5.    The process of Claim 4 wherein the metal hydroxide is sodium hydroxide.

6.    The process of Claim 1 wherein the amount of water present in the mixture is maintained between about 1 and 4% by weight.

7.    The process of Claim 1 wherein the hydroxide is dissolved in a phenol and acetone mixture.

8.    The process for neutralization of the crude phenol-acetone reaction product mixture produced by the cleavage of cumene hydroperoxide by heating in the presence of sulfuric acid characterized by adding to the mixture a neutralizing amount of a metal hydroxide selected from lithium, sodium, potassium, rubidium, cesium, calcium, stronium and barium dissolved in a phenol and acetone solvent, said hydroxide being present at a concentration of about one-fourth to four times the normality of the sulfuric acid in the mixture, controlling the pH of said mixture between about 2 and 7, regulating the temperature between about 20°C and the boiling point of the mixture, maintaining the amount of water present in the mixture at less than about 6% by wt and removing the resultant precipitated sodium sulfate salt as formed.

9.      The process of Claim 8 wherein the temperature is between about 50°C and boiling point of the mixture.

10.      The process of Claim 8 wherein the concentration of hydroxide is about equivalent to the concentration of sulfuric acid in the mixture.

11.      The process of Claim 10 wherein the hydroxide is sodium hydroxide.

12.      The process of Claim 8 wherein the pH of the mixture is between abour 3 and 5.

13.      The process of Claim 8 wherein the amount of water present in the mixture is maintained between about 1 and 4% by weight.

14.      The process for the production of alkyl aromatic hydroperoxide rearrangement products comprising cleaving alkyl aromatic hydroperoxides by heating in the presence of an inorganic acid catalyst to produce a crude product mixture characterized by adding to said mixture a neutralizing amount of a metal hydroxide selected from lithium, sodium, potassium, rubidium, cesium, calcium, strontium and barium, controlling the pH of the crude product mixture between about 2 and 7, regulating the temperature of the crude product mixture between about 20°C and the boiling point of the mixture, maintaining the water content of the crude product mixture at below 6% by weight, removing the resultant precipitated inorganic salt as formed and separating the rearrangement products from the neutralized crude product mixture.


15.    The process for the production of phenol and acetone
comprising cleaving cumene hydroperoxide by heating in the presence
of sulfuric acid catalyst to produce a crude phenol and acetone
mixture characterized by adding to said mixture a neutralizing amount
of sodium hydroxide dissolved in a phenol and acetone mixed solvent
at a concentration about equivalent to the concentration of acid
catalyst in the mixture, controlling the pH of said mixture between
about 3 and 5, regulating the temperature of said mixture between
about 20°C and the boiling point of the mixture, maintaining the
water content of said mixture between about 1 and 4% by weight, re-
moving the resultant precipitated sodium sulfate as formed and
separating phenol and acetone from the neutralized crude phenol and
acetone mixture.

## EUROPEAN SEARCH REPORT

**European Patent Office**

**0085289**
Application number

EP 82 87 0080

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | US-A-2 789 142 (A.R. GRAHAM) <br> * Claims 1, 2 * <br><br> --- | 1,2,5 | C 07 C 37/08 <br> C 07 C 45/53 <br> C 07 C 39/04 <br> C 07 C 49/08 |
| X | US-A-3 284 506 (A. MANTAGAZZA et al.) <br> * Claim 1 ; examples 1, 2 * <br><br> --- | 1,5,7 | |
| A | US-A-4 112 244 (E.N. NOWAK et al.) <br> * Claim 1, position B * <br><br> --- | 1,5 | |
| A | US-A-4 119 791 (W.S. HOLLINGSHEAD et al.) <br> * Claim 1, position B * <br><br> ----- | 1,5 | |
| | | | TECHNICAL FIELDS SEARCHED (Int. Cl. ³) <br><br> C 07 C 37/08 <br> C 07 C 39/04 <br> C 07 C 45/53 <br> C 07 C 49/08 |

The present search report has been drawn up for all claims

| Place of search | Date of completion of the search | Examiner |
|---|---|---|
| BERLIN | 18-03-1983 | KNAACK M |